# EUROPEAN PATENT APPLICATION

(11) **EP 1 867 988 A1**
(43) Date of publication of application: **19.12.2007**
(21) Application number: 06012034.2
(22) Date of filing: 12.06.2006
(51) Int. Cl.: G01N 33/50, G01N 33/68

(54) **Cytokine capture assay in high throughput format for determining antigen-specific T-cell responses**

(71) Applicant: Centro Biotecnologie Avanzate, 16132 Genova (IT); Istituto Giannina Gaslini, 16148 Genova (IT)
(72) Inventor: Manca, Fabrizio, Largo G. Gaslini 5 16148 Genova (IT); Li Pira, Giuseppina, Largo Rosanna Benzi 10 16132 Genova (IT)
(74) Representative: Banfi, Paolo

(57) **Abstract**

The invention provides a method for determining antigen-specific T cell response in a microarray system consisting of microwells wherein a T-cell preparation, e.g. a PBMC or lymphocyte sample from lymphopenic or pediatric patients containing from 5000 to 50000 cell per well, is challenged with the antigen and the cytokines thereby released are detected with an immunoassay, e.g. ELISA.

## Description

The present invention provides a method for determining antigen-specific T cell response in a microarray system. More specifically, the antigen-specific T cell response is assayed in a microwell wherein a cell preparation containing T-cells is challenged with the antigen and the cytokines thereby released are detected with an immunoassay. The microarray approach according to the invention allows testing of cellular immunity in blood samples from lymphopenic or pediatric patients. Thanks to its simplicity, low cost and rapid readout, this assay can be conveniently applied to high throughput screening of compounds modulating the cellular immune response and used as a field test in developing countries for the monitoring of vaccine trials.

### Background of the invention

The analysis of cellular immune responses to antigenic proteins derived from pathogens, tumors, allo or autoantigens is a valuable tool for basic research and for clinical applications (1). Epitopes can be mapped on immunogenic proteins, thus providing information that can be applied to the design of peptide based vaccines or to the development of reagents for diagnostic purposes like MHC-peptide multimers or peptide libraries. Different methods have been developed in the past and more recently flow cytometry based techniques have allowed the enumeration of antigen specific T cells that respond to a given antigen.

A major limitation of these assays is that on average 500 - 200 ×10³ peripheral blood mononuclear cells (PBMC) are needed to test one single antigen. This means that a 10 ml blood sample from a healthy individual, that on average yields 10×10⁶ PBMC, can be used to assess about 50 antigenic samples (e.g peptides). In case blood samples from lymphopenic patients (the populations in which more frequently an assessment of cellular immunity is required) are to be analyzed, the number of antigens that can be tested decreases accordingly. In the case of pediatric samples, the small blood volumes may also limit the analytical breadth. Scaling down the assay format may help overcome these limitations. It should be kept in mind, though, that scaling down has a threshold in the number of cells to be challenged with one single antigen, that is imposed by the frequency of the specific T cells. It is fair to state that the range of memory T cells that can be reasonably detected is between 2% (high frequency) and 0.1% (low frequency). Therefore, in the latter case, at least 10³ PBMC must be analyzed to be confident that at least one specific cell is present and can be detected. To stay on the safe side, we should examine at least 5×10³ PBMC. This is the lowest threshold we should accept.

Several recent publications have addressed the issue of epitope mapping by using a microarray approach (2,3,4). In these cases, purified MHC molecules were spotted on chips and incubated with binding peptides to produce MHC-peptide complexes to simulate the surface of an antigen presenting cell on the solid phase. These MHC-peptide complexes were recognized by the corresponding T cell receptors and were able to bind and retain specific T cells. Binding of pre-labeled fluorescent cells (2) and in situ identification of secreted cytokines were used as readouts (3,5). These techniques are certainly appealing, but at the present time are not applicable in the clinical diagnostic setting for the following reasons: 1. sophisticated and expensive instrumentation is needed to spot the MHC molecules, dispense the synthetic peptides and finally read the microchips. 2. since few human MHC alleles are available, only T cell repertoires restricted to these alleles can be analyzed. 3. the subject to be examined needs to be HLA typed. 4. HLA class I alleles are used predominantly, as information on HLA class II alleles for formation of MHC-peptide complexes recognized by CD4 T helper cells lags behind the MHC class I technique, as in the case of class I vs class II tetramers. 5. presentation of antigenic peptides is artificial and the high density of MHC-peptide molecule, with most MHC molecules presenting one selected peptide, may not mirror the APC surface where the relevant peptide is diluted among a host of irrelevant peptides bound to MHC; therefore also T cells with low or physiologically irrelevant affinity, in principle may bind and give positive signals. 6. antigens that need processing by APC cannot be tested, therefore this screening cannot be applied to proteins. 7. finally, even if chip technologies are used, we still have to consider the lowest threshold of 5-10×10³ PBMC to be challenged with one single antigen for the reasons we have discussed above.

An additional method based on microarrays of single cells has been reported recently (5). In this case nanowells are constructed so to accommodate one single cell. B lymphocytes are examined in this case. After contact with antigen, specific B cells are activated and revealed by fluorescence based methods that detect calcium influx. This method, though, is not appropriate for testing cellular immune responses mediated by T lymphocytes, that require antigen presenting cells (APC) for stimulation. In addition, this assay based on analysis of one cell/well requires that at least 1000 thousand wells are seeded and scanned for each single antigen. Also in this case the complexity of the system and the need for expensive equipment make this approach unfeasible for clinical-diagnostic applications and for high throughput epitope mapping of T cell immunity.

Therefore there is an urgent need for the development of high throughput assays for cellular immunity that make use of much smaller numbers of PBMC. Obviously, scaling down molecular assays has practically no limits, while scaling down lymphocyte culture conditions has three major limitations. First, handling of functional microcultures in adequate containers poses technical challenges. Second, the readout system for T cell responses must be simple, specific, sensitive, miniaturized and reproducible. Third, the frequency of antigen specific cells prevents unlimited scaling down. In fact, assuming a reasonable frequency of 0.1% T cells specific for a given recall antigen, cultures must contain more than 1000 cells to have at least one single cell per well giving a positive signal when stimulated with antigen.

### Description of the invention

It has been found that an effective, highly-sensitive determination of antigen-specific T cell response can be performed in a high troughput format, using cultures of as few as 5×10³ to 50×10³ cells dispensed in microwells with 5 to 50 µl capacity.

Accordingly, the invention provides a method for determining antigen-specific T cell response in a high throughput format, which comprises the following steps:
i) providing a multiwell plate suitable for culturing cells in a volume of from 5 to 50 µl/well;
ii) coating the wells with anti-cytokine antibodies;
iii) adding the test antigen to the wells;
iv) providing a PBMC (peripheral blood mononuclear cells) or lymphocyte culture and dispensing it in the wells in amounts of from 5×10³ to 50×10³ PBMCs or lymphocytes per well ;
v) incubating the cells for a time sufficient for antigen-mediated T cell stimulation to occurr;
vi) detecting the presence of the cytokine secreted in the culture medium and immobilized by the anti-cytokine antibody of step ii), by means of an immunoassay.

In a preferred embodiment the assay is carried out in a 384 or 1536 well plate and the PBMCs or the lymphocytes are preferably separated from human blood samples. Because of the use of very few cells in comparison to current methods, this is ideal for lymphopenic patients or for small volume samples (pediatric patients). In this system, autologous APC are present in the PBMC preparation. Monocytes, B cells, dendritic cells expressing HLA class II alleles can present to CD4 lymphocytes either proteins that require processing or peptides that directly bind to class II molecules. All mononucleated cells expressing HLA class I molecules (T and B lymphocytes, monocytes, dendritic cells, NK cells) are adequate for direct presentation of class I restricted peptides. For more accurate simulation of class I restricted presentation to CD8 cells, virus infected cells can also be added to the wells, while this is not feasible with MHC-peptide chip arrays.

According to the invention, the antigens may include HLA class I or class II restricted peptides, proteins, virus, virus-infected cell, cell- or virus lysate, for example tetanus toxin, PPD (M. bovis), CMV pp65 and IE-1 protein, CMV lysate, T. gondii lysate, lysates of P. carinii, Aspergillus sp. and cryptococcus cells, SARS coronavirus spike protein peptides, HIV and HTLV1 peptides and proteins, selected immunodominant peptides from adenovirus, respiratory syncytial virus and other relevant viruses.

Cytokines are rapidly synthesized by specific T cells upon antigen recognition. According to the invention, the cytokines preferably are IL2, IL4, IL5, IFNg, TNFa and GM-CSF. In order to have a cytokine production in detectable amounts, the T cells are generally incubated in the presence of the antigens for a time period of 18-24 hrs.

In a preferred embodiment of the invention, the cytokines secreted in the culture medium and immobilized by the anti-cytokine antibody are detected by ELISA. The latter preferably comprises the following steps:
i) contacting a labeled antibody with the antibody-cytokine complex, to form an antibody-cytokine-labeled antibody complex;
ii) washing;
iii) contacting the complex formed in step i) with a labeled enzyme able to produce a detectable signal upon reaction with the substrate, to form a antibody-cytokine-labeled antibody-labeled enzyme complex;
iv) washing;
v) contacting the enzyme in the complex formed in step iii) with the substrate;
vi) detecting the signal produced by the enzyme-substrate reaction.

Preferably the antibody used in step i) is biotinylated and the labeled enzyme used in step iii) is alkaline phosphatase conjugated streptavidin or streptavidin conjugated to other convenient enzymes (e.g. peroxidase, beta-galactosidase).

This assay can be be applied to large scale screening of peptide panels to identify peptides that activate T cells, irrespective of the individual cytokines that are produced. In particular, by using the so called polyELISA test, positive supernatants that contain anyone of the cytokines revealed by the polyELISA test can be detected. This identifies "cytokine positive wells". If supernatants have been saved in replica plates, selected positive samples can be subsequently examined for individual cytokines in miniaturized formats (microELISA, Luminex assays, cytokine arrays).

It was observed that the ratio between positive response in the presence of antigen and negative response (background in the absence of antigen) is higher than in conventional proliferation assays and in intracytoplasmic staining. This enhanced differential suggests that cell ELISA is more efficient to reveal low responses, in comparison to other current methods.

With respect to sensitivity, the IFNg produced by a single T cell can be detected by titrating a T cell line into non responding PBMC (1 T cell out of 50,000 PBMC, corresponding to a frequency of 0.002%). Limiting dilution experiments confirmed that one single cell can be identified based on Poissonian distribution of the statistically significant positive signals. Based on limiting dilution experiments with specific T cell lines or on titrations of PBMC containing specific CD8 T cells defined by tetramer enumeration, IFNg produced by one single cell in 24 hours ranges between 0.5 and 5 pg.

The method of the invention is particularly suitable for:
1. basic research: epitope mapping, definition of cytokine profiles by microcultured cells (e.g. cloned cells) or by selected cell subsets. Specificity screening for antigens / peptides derived from pathogens, tumor antigens, autoantigens, alloantigens
2. clinical investigation: epitope mapping in infected patients, in vaccinated subjects enrolled in vaccine trials, in tumor patients, in transplant recipients, in patients suffering from autoimmune diseases.
3. diagnostic applications: definition of cellular immunity (immunocompetence) specific for a broad range of relevant pathogens. By using minute amounts of cells, the test is particularly suitable for lymphopenic patients and pediatric patients.
4. diagnostics in developing countries: since the assay is simple, inexpensive, fast and requires little technical training, cellular immune responses to poverty related infections like HIV, malaria, TB, HCV, etc can be easily detected and monitored by using pre-coated plates with pre-dispensed antigens in the field.
5. industrial applications: high throughput screening of compounds with known or unknown functions to test their interference with cellular immune responses (immunomodulators). This may lead to the discovery of new immunosuppressive or immunoenhancing compounds, since screening of thousands of compounds can be easily performed with limited cost and in a short period of time in the 1536w format.
6. veterinary applications: by using anti human antibody pairs cross reacting with the species under study, or species-specific antibody pairs, it is possible to screen cellular immunity in a variety of animal species, including primates (also used for basic research) or farmed animals that may be affected by epidemic diseases. The diagnosis of cellular immunity may provide earlier information than serology in viral daseases, and essential information when antibody responses are not significant (e.g. mycobacterial infections).

The attached Figures and the section "Materials and Methods" illustrate the invention in greater detail.

### Description of the Figures

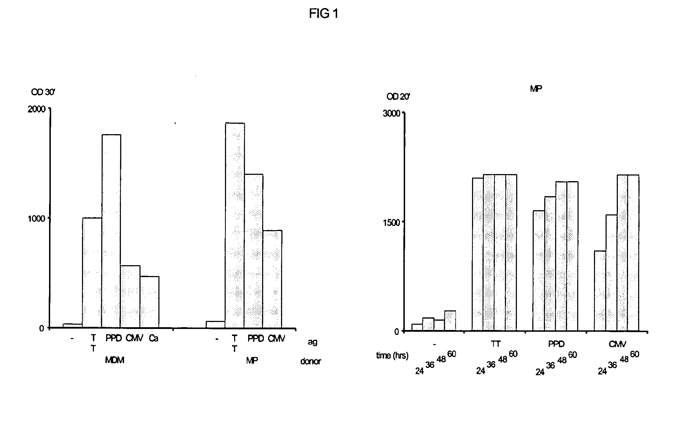

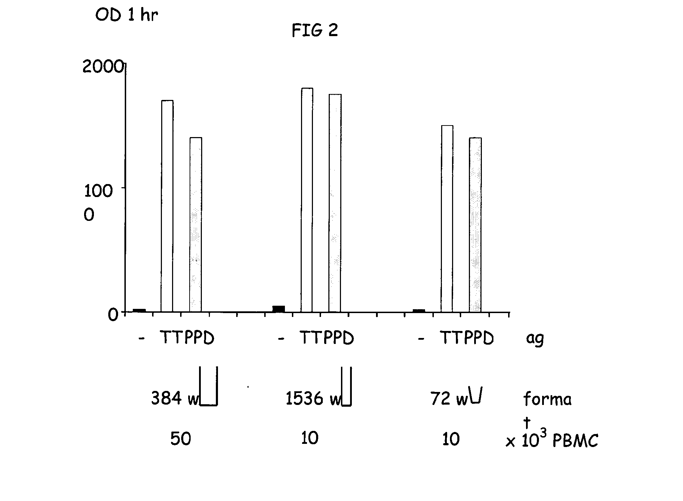

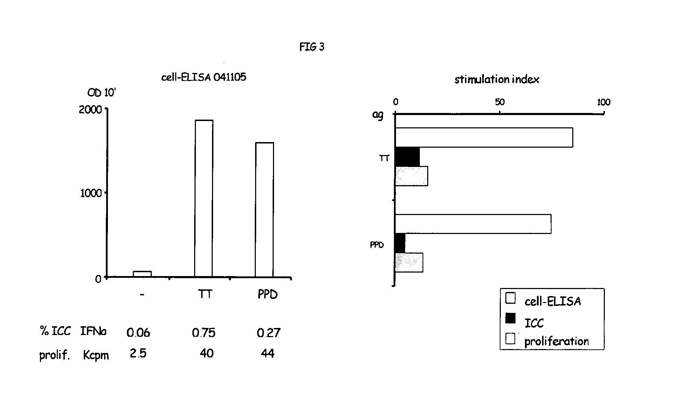

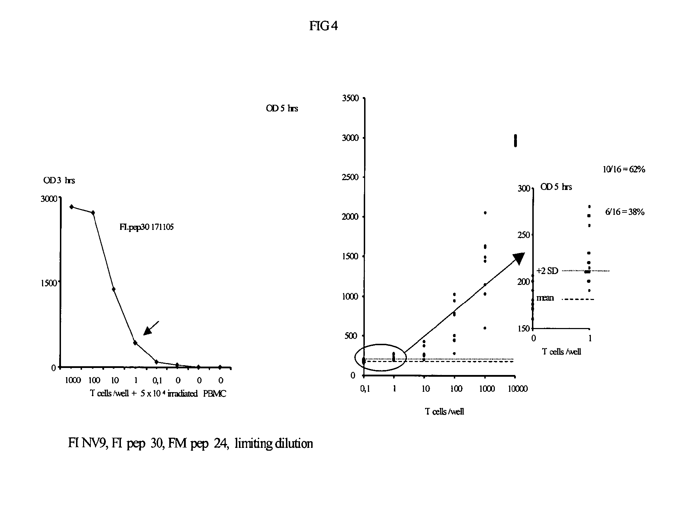

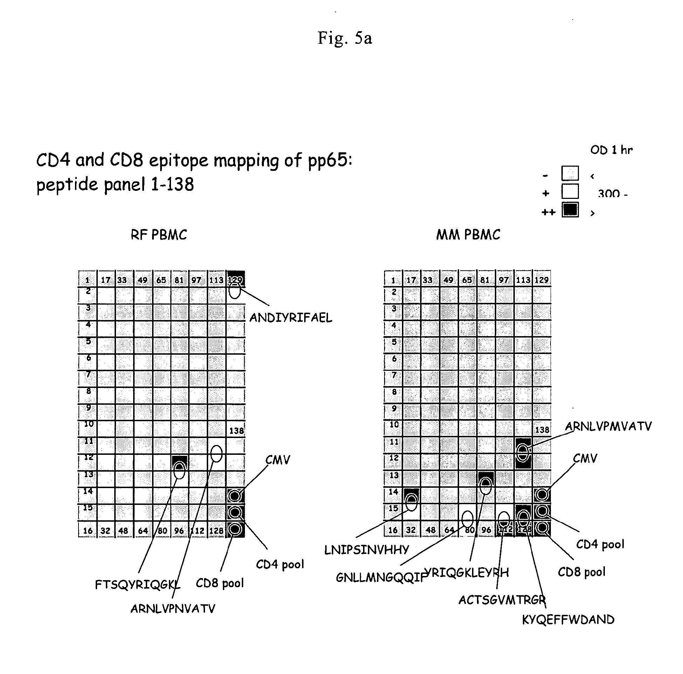

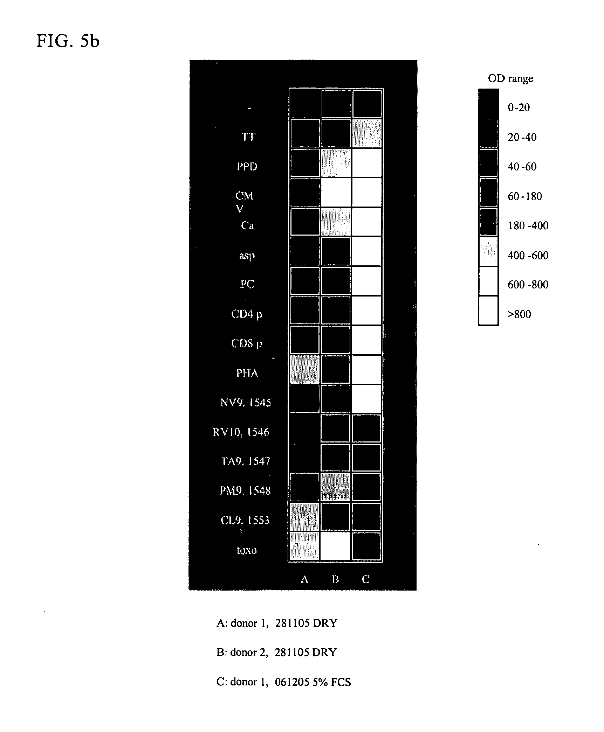

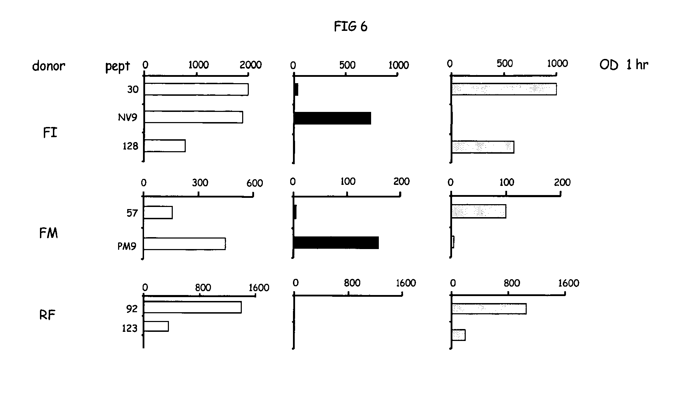

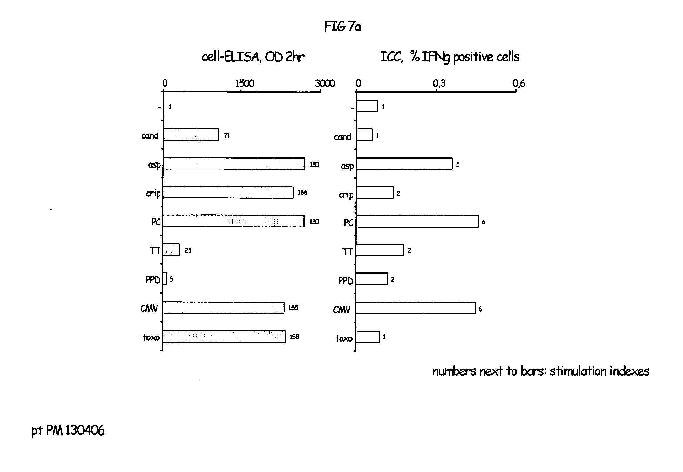

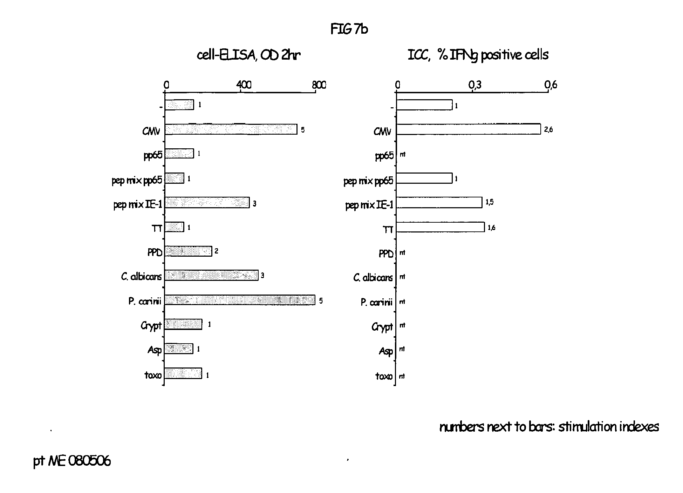

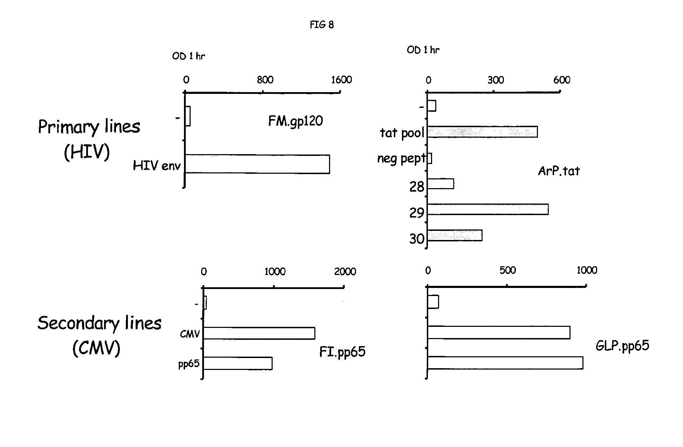

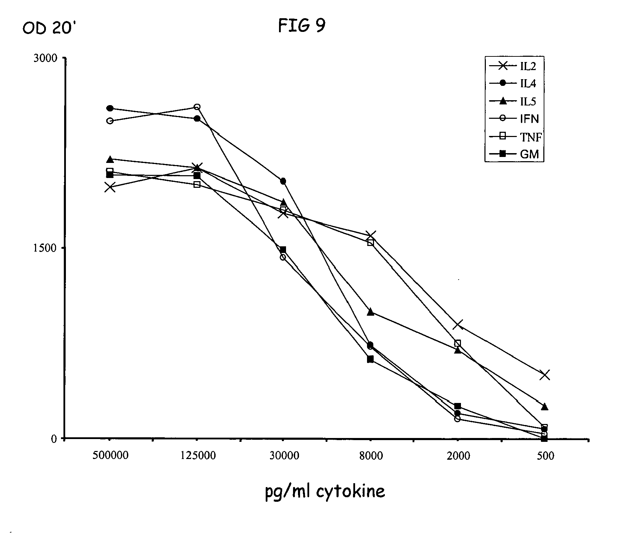

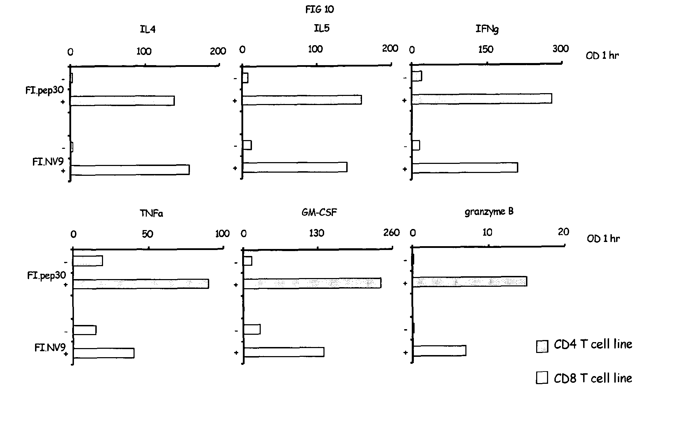

**Fig. 1**
   cellELISA, left panel: IFNg production by PBMC upon antigen stimulation, 24 hrs,
   PBMC (50×10³/well in 50 µl) from donors MDM and MP were cultured in a 384w plate precoated with a-IFNg antibodies, in the presence of different recall antigens. After 24 hrs, sups were removed and wells were developed for ELISA.
   cellELISA, right panel: kinetics of IFNg production.
   PBMC cultures were set up with donor MP as described in Fig. 3. Supernatants were removed at the indicated times and wells were filled with PBS-Tween. After 60 hrs, all wells were developed for ELISA.
   It can be seen a slight but gradual increase in background, while IFNg induced by antigen stimulation (TT, PPD, CMV) reaches a plateau at 36-48 hrs. For convenience, an incubation time of 20-24 hrs was chosen in subsequent assays.
**Fig. 2**
   Use of 1536w plates for culture and cellELISA.
   In order to further scale down the culture volumes, we run assays in 1536w plates, that received antigens in 1 µl volume and cells in 10 µl volume (10e4 PBMC/well). The same conditions were used to seed 72 well plates (Terasaki plates) or 384 well plates with conical shape wells that take up to 20 µl working volume. After culturing and processing the wells as described, the developed substrate PNPP was harvested from four replicate wells from the 1536w plate and read as 40 µl in a 384w plate (in fact we do not have a 1536 plate reader available at the present time). The figure shows that excellent results can be achieved also in these conditions, indicating that both cultures and ELISA assays can be scaled down to the 1536w format or to the 10 µl volume.
**Fig. 3**
   Comparison of cellELISA with standard methods for evaluation of specific T cell responses: cellELISA vs intracytoplasmic IFN staining vs proliferation.
   CellELISA and ICC were tested after 24 hrs, proliferation after 5 days. Results are given as OD, % positive cells and Kcpm respectively. Stimulation indexes for the different assays are shown on the right.. The highest indexes, indicating the highest differential between unstimulated and stimulated PBMC, were obtained by cellELISA.
**Fig. 4**
   Sensitivity of cellELISA.
   CD4 T cell line specific for CMV pp65 peptide 30 (117-131, PLKMLNIPSINVHHY) (containing 100% specific cells according to ICC after > 6 restimulation cycles with antigen and APC) was spiked into autologous irradiated (non responding) PBMC. Six replicate wells were set up for each T cell dilution. Results are shown as average value of replicates. Background response of irradiated PBMC to pep30 (120 OD) was subtracted. It can be seen that 1 cell/well still yields a positive signal, corresponding to detection of 1 specific cell out of 50,000 PBMC (1/50,000 = 0.002%).
   Limiting dilution analysis.
   The CD4 T cell line was diluted into autologous irradiated PBMC so to have 1000, 100, 10, 1 cell per well in 16 replicates, plus 50×10^{∧}3 PBMC per well as APC. Positive wells were defined as those giving a signal higher than the mean value of negative background wells (receiving no T cells) + 2 SD (210 OD). All wells with 10 or >10 specific T cells were positive. Among the wells with 1 specific T cell, 62% were positive and 38% were negative. This matches the expected theoretical frequency of 63% positive and 37% negative for a single hit event.
**Fig. 5**
   **5a.** Antigen arrays: protein antigens and peptide panels
      PBMC from different donors were cultured with various antigens (tetanus toxoid, PPD, CMV, C.albicans, Asp.niger, P.carinii, pool of CMVpp65 Th peptides, pool of CMV pp65 CTL peptides, PHA, CMV peptides (NV9=A2, TA9-CL9=B7), T. gondii). Representative results are shown in 5a in an array format, with shades corresponding to OD ranges (left panel).
   **5b.** Two donors were also screened with a paenel of overlapping peptides that encompas the immunodominant protein pp65 of CMV (5b). The identified stimulatory peptides fully matched with the information we already had from those donors with respect to their fine specificity profiles.
**Fig. 6**
   CD4 and CD8 responses
   Our assay format on whole PBMC does not discriminate between CD4 and CD8 responses. Therefore we demonstrated that by simple, one step removal of one of the two subsets with magnetic beads (Dynabeds, Dynal, Denmark), we can achieve a clear-cut discrimination. Three representative subjects are shown here. The left graphs show responses of unfractionated PBMC to a set of immunodominnat CD4 or CD8 peptides for the various donors. As predicted, CD4+ cells (middle panels) only responded to CD4 peptides, but not to CD8 peptides, and viceversa (right panels).
**Fig. 7**
   Examples of diagnostic applications
   Two children from whom small amounts of blood were available were screened for cellular immunity against a panel of relevant antigens. Informative responses were obtained by cellELISA in 384 well plates, while conventional intracytoplasmic staining (ICS) for IFNg was occasionally negative and showed much lower stimulation indexes. As frequently occurrs with pediatric samples, the number of PBMC was not sufficient to run an extensive screening by ICS with the second patient (7b), while the miniaturized format allowed the complete screening with the panel of recall antigens.
**Fig. 8**
   Use of cellELISA to screen T cell lines
   In the research setting, our assays provides information also on responsiveness and specificity of established T cell lines.The major advantage over convential lymphoproliferation used for these studies is that no radioactivity is used, very few cells are needed and the result can be available in 24 hrs instead of 3-4 days. The figure shows experiments with T cell lines derived from naive precursors specific for HIV (upper panels) and T cell lines specific for recoll antigens (pp65 of CMV).
**Fig. 9**
   Validation of the polyELISA test
   Wells were coated wityh a pool of 1 st antibodies specific for IL2, IL4, IL5, IFNg, TNFa, GM-CSF. Individual recombinant cytokines were titrated. A pool of 2nd biotinylated antibodies with the same specificities was added, followed by streptavidine-alkaline phosphatase and substrate. The individual titration curves show that the assay can reveal individual cytokines, without interference among the different antibody-cytokine systems.
**Fig. 10**
   Screening of polyELISA positive supernatants for individual cytokines
   Supernatants from a CD4 T cell line (pep30) and from a CD8 T cell line (NV9) specific for immunodominant peptide of CMV were tested by polyELISA (not shown). The positive supernatants were tested for individual cytokines, as shown here. This confirmas that polyELISA can detect either single cytokines (fig 9) or mixture of cytokines naturally produced by antigen-activated T cell.

### Materials & Methods

### 1. Reagents

### 1.1 Antigens

Proteins: tetanus toxoid (TT) and PPD were purchased from Statens Seruminstitut, Copenhagen, Denmark; CMV pp65 protein expressed in E.coli was provided by A. Loregian, University of Padua IT; CMV lysate and T. gondii lysate were purchased from ABI, Columbia, MD; P. carinii suspension was provided by J-C. Caillez, Pasteur Institute, Lille, FR; C.albicans, Asp. fumigatus and cryptococcus were prepared in our lab as an autoclaved suspension. Peptides of CMV pp65 and IE-1 proteins, of SARS coronavirus spike protein, of different HIV proteins and selected peptides from other cviruses were synthesized as 15mers overlapping by 11 aa residues when used as comprehensive peptide panels.

### 1.2 ELISA antibody pairs

Ab pairs from Mabtech, Stockholm SE, were used at the indicated dilutions. The kits contain the first antibody for coating, the second biotinylated antibody and alkaline phosphatase conjugated streptavidin. Standards were provided with the kits and prepared as instructed.

### 2. Cells

### 2.1 PBMC

PBMC preparation by standard Ficoll gradient, resuspended at 10⁶/ml in RPMI 5% FCS.

### 2.2 T cell lines

Human CD4 and CD8 T cell lines with different specificities were available in our laboratory. The lines have been produced as described in detail (7,8)

### 3. Materials

Plates were purchased from Nunc, Denmark. 96w plates were either flat bottom or round botton for cultures, Immuno-modules were used for ELISA assays in 96 well format. 384 well and 1536 well plates were clear plastic tissue culture grade. They were used both for cell cultures and for ELISA assays.

### 4. Instrumentation

For reading the ELISA plates, we used the SLT 340 ATL, Tecan, Germany for 96w and the Genios, Tecan for 384w. Since 1536w reader was not yet available, assays in 1536w plates were run as quadruplicates and four wells containing 10 µl PNPP were pooled after incubation in one well of a 384w plate. Results are shown as OD405 × 1000 at the indicated times of incubation at RT.

For sample handling, eight channel electronic pipets (Eppendorf) were used to transfer cells or ELISA reagent in all plate formats. Multiple six channel Hamilton syringes with teflon coated plungers (gas tight) were used with a manual dispense when 1 µl samples (antigens) were distributed in 1536w plates.

A multiwell automatic dispenser (WellMATE, Matrix, Hudson, NH) was used for processing of the 384w plates.

### 5. Methods

### 5.1 Lymphocyte cultures

PBMC prepared by conventional Ficoll gradient were resuspended in RPMI1640 medium containing L-glutamin and 5% fetal calf serum (FCS) or in 5% human AB serum selected for low background in the ELISA assays. Preliminary tests, in fact, showed that sera from certain healthy subjects have detectable concentrations of several cytokines that interfere with the ELISA assay by resulting in a high background. Antigens were prepared as 10x solutions and dispensed in wells before seeding PBMC. PBMC were brought to 10⁶/ml and dispensed at the indicated volumes by using multichannel pipets (200 µl for 96w and 50 µl for 384w plates) or Hamilton syringes (10 µl for 1536w plates). Plates were incubated overnight, supernatants were transfered to replica plates for later use and the culture plate was developed for cellELISA.

### 5.2 ELISA assays

The volumes of the reagents in the different plate formats are summarized below:

| reagent | 96 | 384 | 1536 |
|---|---|---|---|
| I ab, 1 µg/ml in PBS | 100 | 20 | 5 |
| saturation, PBS HSA 1% | 200 | 100 | 10 |
| sample | 200 | 50 | 10 |
| II ab, 1 µg/ml in PBS HSA 1% | 100 | 20 | 5 |
| Strep-ALP, 1:200 in PBS HSA 1% | 100 | 20 | 5 |
| PNPP | 200 | 50 | 10 |

| | | | |
|---|---|---|---|
| Coating with I ab, ON, RT, washing with PBS 4x (flushing with wash bottle) Saturation with HSA 1% in PBS, 1 hrs, washing PBS 4x (flushing with wash bottle) Sample, 1 hr, washing with PBS 0.05% Tween 2x, PBS 4x (flushing with wash bottle) II ab, 2 hrs, washing with PBS 0.05% Tween 2x, PBS 4x (flushing with wash bottle) Strep-ALP, 1 hr, washing PBS 0.05% Tween 2x, PBS 4x (flushing with wash bottle) PNPP, as needed (30' - 6 hrs), read OD at 405 nm | | | |

### 5.3 PolyELISA

Coating antibodies at 1 mg/ml specific for different cytokines (IL2, IL4, IL5, IFNg, TNFa, GM-CSF, 20 µl each) were pooled. For coating, 6 µl of the pool were added to 1 ml PBS. Biotinylated antibodies specific for the same set of cytokines (20 µl each) were pooled. When used as second reagent, 2 µl of this pool were added to 1 ml PBS 1% HSA. ALP-streptavidin was used at 1:2000 as indicated.

### 5.4 CellELISA

Culture wells (96, 384 or 1536 well plates) were handled under sterile conditions for coating with sterile I antibody. Ofter ON coating and 1 hr saturation with HSA 1% in PBS, the liquid was removed by aspiration with a blunt needle connected to a vacuum source. Antigens as 10× solutions were dispensed in the plates (10 µl in 96 well plates, 5 µl in 384, 1 µl in 1536) and let to dry for 1 hr under the laminar flow cabinet. The plates were coated with an adherent plastic sheet and stored at room temperature. PBMC were dispensed at the indicated volumes. After ON incubation, supernatants were transfered to a replica plate and the wells were developed.

### 6. T cell lines as standards

T cell lines with CD4 or CD8 phenotype and specific for Tetanus Toxoid (TT), HIV env and several epitopes of CMV pp65 have been used as standards to run sensitivity experiments. By using established T cell lines (7,8), in fact, we know exactly the number of input antigen specific T cells. Thus we can run limiting dilution experiments that are not feasible with PBMC, in which the actual number of specific cells can only be inferred by assays like tetramers, ELISPOT or intracytoplasmic cytokine staining, assuming 100% efficiency.

### REFERENCES

1. Kern F. Li Pira G, Gratama J. Manca F, Roederer M. Measuring antigen specific immune responses: understanding immunopathogenesis and improving diagnostics in infectious diseases, autoimmunity and cancer. Trends Immunol. 2005,9,477-484.
2. Soen Y, Chen DS, Kraft DL, Davis MM, Brown PO. Detection and characterization of cellular immune responses using peptide-MHC microarrays. Plos Biol. 2003,1,429-438.
3. Stone JD, Demkowicz WE, Stern LJ. HLA-restricted epitope identification and detection of functional T cell responses by using MHC-peptide and costimulatory microarrays. PNAS 2005,102,3744-3749.
4. Toebes M, Coccoris M, Bins A, Rodenko B, Gomez R, Nieuwkoop NJ, van de Kasteele W, Rimmelzwaan G, Haanen JB, Ovaa H, Schumacher TN. Design and use of conditional MHC class II ligands, Nature Med. 2006, 121,246-250.
5. Chen D, Soen Y, Stuge TB, Lee PP, Weber JS, Brown PO, Davis MM. Marked differences in human melanoma antige-specific T cell responsiveness after vaccination using a functional microarray. PLoS Medicine 2005,2, 1018-1030.
6. Yamamura S, Kishi H, Tokimitsu Y, Kondo S, Honda R, Rao SR, Omori M, Taliya E, Muraguchi A. Single cell microarray for analyzing cellular response. Anal. Chem. 2005,77,8050-8056.
7. Li Pira G, Bottone L, Ivaldi F, Del Galdo F, De Palma R, Manca F. Human naive CD4 T cell clones specific for HIV env persist for years in vivo in the absence of antigenic challenge. J. AIDS, 2005,40,132-139.
8. Li Pira G, Bottone L, Ivaldi F, Manca F. Generation of Cytomegalovirus specific CD4 T cell lines devoid of alloreactivity using a cocktail of CMV-pp65 peptides for reconstitution of the Th repertoire. J. Infect. Dis., 2005,191,215-226.
9. Li Pira G, Bottone L, Ivaldi F, Einsele H, Palù G. Identification of new Th peptides from the cytomegalovirus immunodominant protein pp65 and use as a peptide library for generation of T cell lines for cellular immunoreconstitution. Int. Immunol. 2004, 16, 635-642.

## Claims

1. Method for determining antigen-specific T cell response in a high throughput format, which comprises the following steps:
i) providing a multiwell plate suitable for culturing cells in a volume of 5 - 50 µl/well;
ii) coating the wells with anti-cytokine antibodies;
iii) adding the test antigen to the wells;
iv) providing a PBMC (peripheral blood mononuclear cells) or lymphocyte culture and dispensing it in the wells in amounts of 5×10³ - 50×10³ cells per well;
v) incubating the cells for a time sufficient for antigen-mediated T cell stimulation to occurr;
vi) detecting the presence of the cytokine secreted in the culture medium and immobilized by the anti-cytokine antibody of step ii), by means of an immunoassay.

2. A method according to claim 1, wherein the multiwell plate is a 384 (50 µl culture volume) or 1536 (5-10 µl culture volume) well plate.

3. A method according to claim 1, wherein the antigen is a class I- or a class II-restricted epitope.

4. A method according to claim 3, wherein the antigen is a peptide, protein, virus, virus-infected cell, cell- or virus lysate.

5. A method according to claim 4, wherein said antigen is selected from tetanus toxin, PPD (M.bovis), CMV pp65 and IE-1 protein peptides, CMV lysate, T. gondii lysate, lysates of P. carinii, Asp. species and cryptococcus cells, SARS coronavirus spike protein, HIV peptides.

6. A method according to claims 3-5, wherein different antigens are tested in the same multiwell plate.

7. A method according to claim 1, wherein the detected cytokine is IL2, IL4, IL5, IFNg, TNFa, GM-CSF.

8. A method according to claim 1, wherein in step v) the cells are incubated for a period of 18 - 24 hrs.

9. A method according to claim 1, wherein the immunoassay of step vi) is ELISA.

10. A method according to claim 9, wherein the ELISA is carried out by:
i) contacting a labeled antibody with the antibody-cytokine complex, to form an antibody-cytokine-labeled antibody complex;
ii) washing;
iii) contacting the complex formed in step i) with a labeled enzyme able to produce a detectable signal upon reaction with the substrate, to form a antibody-cytokine-labeled antibody-labeled enzyme complex;
iv) washing;
v) contacting the enzyme in the complex formed in step iii) with the substrate;
vi) detecting the signal produced by the enzyme-substrate reaction.

11. A method according to claim 10, wherein the labeled antibody used in step i) is a biotinylated antibody.

12. A method according to claim 10, wherein the labeled enzyme used in step iii) is alkaline phosphatase conjugated streptavidin, peroxidase or beta-galactosidase conjugated streptavidin.

13. A method according to any preceding claim, wherein PBMCs and lymphocytes are separated from isolated human body fluid samples.

14. A method according to claim 13, wherein the PBMC or lymphocytes are separated from the blood of lymphopenic or pediatric human patients.

15. A method according to claim 13, wherein the PBMC or lymphocytes are taken from the blood of non-human animals.
